(19) Europäisches Patentamt
European Patent Office
Office européen des brevets

(11) **EP 3 120 770 A2**

(12) **EUROPEAN PATENT APPLICATION**

(43) Date of publication:
**25.01.2017 Bulletin 2017/04**

(51) Int Cl.:
**A61B 5/1455** (2006.01)  **G01N 21/359** (2014.01)
**G01N 21/49** (2006.01)

(21) Application number: **16179461.5**

(22) Date of filing: **14.07.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **23.07.2015   JP 2015145609**

(71) Applicant: **Advantest Corporation
Tokyo 179-0071 (JP)**

(72) Inventor: **KIMURA, Makoto
Tokyo, 179-0071 (JP)**

(74) Representative: **Tetzner, Michael et al
TETZNER & PARTNER mbB
Patent- und Rechtsanwälte
Van-Gogh-Strasse 3
81479 München (DE)**

(54) **INFRARED OPTICAL BIOSENSING APPARATUS AND PROBE THEREOF**

(57)    A infrared optical biosensing apparatus is provided which includes: a probe including an infrared light emitting element which emits infrared light, an output variation detection element which detects output variation of the infrared light emitting element, and a light receiving element which detects the infrared light scattered in a living organism; and an operation unit which calculates information on the living organism based on output data of the light receiving element. The operation unit corrects the information on the living organism in accordance with a detection result of the output variation detection element.

FIG. 3

**Description**

[Technical Field]

The embodiments discussed herein are related to a near-infrared optical biosensing apparatus and a probe thereof which emit near-infrared light to a living organism and obtain information on inside of the living organism.

[Background Art]

The near-infrared spectroscopy (NIRS), which emits near-infrared light and detects intensity changes of the light scattered from the inside of a living organism, is receiving attention as a method capable of measuring a blood flow rate and a state of hemoglobin in the brain without damaging the living organism.

A near-infrared optical biosensing apparatus performs measurement with a probe attached to a surface of the living organism. Measurement with enough sensitivity requires the stability of the intensity of the applied infrared light. To this end, the apparatus requires a laser oscillator which is operated under temperature control unit, and the infrared light is guided to the probe through optical fiber.

However, since a thick and heavy optical fiber cable is connected to the probe, the probe imposes a heavy burden on the subject such as a child.

Meanwhile, there has also been proposed an apparatus in which the probe is equipped with an infrared light-emitting diode (infrared LED) with an output stabilizing circuit. In this case, since the output stabilizing circuit is mounted in addition to the infrared LED, the probe becomes larger and heavier, and accordingly imposes a heavier burden on the skin when the probe is attached with adhesive tape.

For this reason, a subject with a small body such as a child, for example, may feel discomfort from the large probe, and behave uncooperatively to an examination to hinder the smooth examination.

    Japanese Laid-open Patent Publication No. 2005-111165
    Japanese Laid-open Patent Publication No. 2006-17084
    Japanese Laid-open Patent Publication No. 2008-178563

[Summary of Invention]

[Technical Problem]

An object of the invention is to provide a near-infrared optical biosensing apparatus and a probe thereof, whose burden on a subject is small.

[Solution to Problem]

According to an aspect of the disclosure below, a infrared optical biosensing apparatus is provided which includes a probe including an infrared light emitting element which emits infrared light, an output variation detection element which detects output variation of the infrared light emitting element, and a light receiving element which detects the infrared light scattered in a living organism; and an operation unit which calculates information on the living organism based on output data of the light receiving element, and in which the operation unit corrects a detection result of the light receiving element in accordance with a detection result of the output variation detection element, and obtains the information on inside of the living organism.

According to another aspect, a probe of a infrared optical biosensing apparatus is provided which includes: an infrared light emitting element which emits infrared light; an output variation detection element which detects output variation of the infrared light emitting element; a light receiving element which detects the infrared light scattered in a living organism; and a drive circuit which supplies drive current to the infrared light emitting element, and in which the drive circuit supplies constant drive current to the infrared light emitting element independently of temperature.

[Advantageous Effects of Invention]

According to the near-infrared optical biosensing apparatus of the above aspects, variation of detection data of the light receiving element caused by output variation of the infrared light emitting element is corrected using the output value of the output variation detection element.

With this configuration, the probe does not have to be equipped with a control circuit for stabilizing output of the infrared light emitting element. This makes it possible to downsize the probe, and provide the near-infrared optical biosensing apparatus whose burden on a subject is small.

[Brief Description of Drawings]

[Fig. 1] Fig. 1 is a graph illustrating spectra of molar absorption coefficients of hemoglobin in a living organism to infrared light.
[Fig. 2] Fig. 2 is a diagram illustrating concentration changes of oxygenated hemoglobin and deoxygenated hemoglobin with changes in a blood flow rate.
[Fig. 3] Fig. 3 is a diagram illustrating a measurement method for a brain function using near-infrared light.
[Fig. 4] Fig. 4 is a diagram illustrating a schematic structure of a near-infrared optical biosensing apparatus according to an embodiment.
[Fig. 5] Fig. 5 is a block diagram of the near-infrared optical biosensing apparatus in Fig. 4.
[Fig. 6] Fig. 6(a) is a plan view illustrating a layout of a light emitting element and light receiving elements of a probe in Fig. 4. Fig. 6(b) is a cross-sectional view illustrating the layout of the light emitting element and the light receiving elements in the probe in Fig. 4.
[Fig. 7] Fig. 7 is a flowchart of a measurement method using the near-infrared optical biosensing apparatus in Fig. 4.
[Fig. 8] Fig. 8 is a flowchart of a measurement process (step S2) in Fig. 7.
[Fig. 9] Fig. 9 is a timing chart illustrating operations of light-emitting diodes, a light receiving element, and a selector.
[Fig. 10] Figs. 10(a) to 10(c) are diagrams each illustrating a layout of an output variation detection element 13 according to a modification.

[Description of Embodiment]

Before describing an embodiment, descriptions are provided for a prelude as basic information.
In a near infrared range (650nm-2500nm), infrared light with a wavelength from 650 nm to 1000 nm has high transmittance through a living organism and is also referred to as a window to living organisms, where the light can reach deep inside of the living organism. When near-infrared light with a wavelength around those of the window to living organisms is applied to a living organism, a part of the light is scattered inside the living organism and returns to the surface.
Detecting intensity change in this scattered light makes it possible to obtain inside information of the living organism such as a change in a blood flow rate in real time without damaging the living organism.

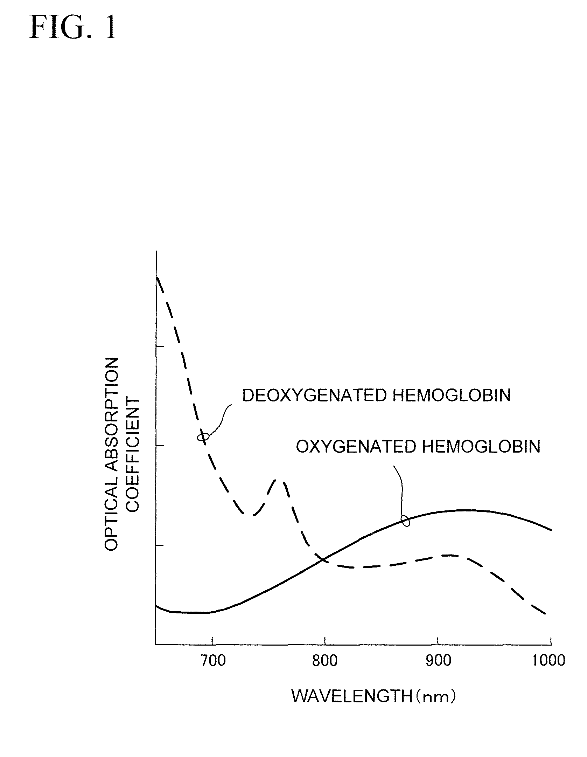

Fig. 1 is a graph illustrating spectra of molar absorption coefficients of hemoglobin in a living organism to infrared light.
Hemoglobin in a living organism includes oxygenated hemoglobin to which oxygen is bound and deoxygenated hemoglobin from which oxygen is released. As illustrated in Fig. 1, oxygenated hemoglobin shows a higher absorption rate to infrared light with a longer wavelength (800 nm or longer) than deoxygenated hemoglobin, whereas the deoxygenated hemoglobin shows a higher absorption rate to infrared light with a shorter wavelength (800 nm or shorter) than the oxygenated hemoglobin.
For this reason, when infrared light with a wavelength around 850 nm is applied to a living organism, the intensity of scattered light is affected dominantly by concentration changes of oxygenated hemoglobin. On the other hand, for infrared light with a wavelength around 750 nm, the intensity of scattered light is affected dominantly by concentration changes of deoxygenated hemoglobin. Using infrared light with two wavelengths makes it possible to find out the concentration changes of both oxygenated hemoglobin and deoxygenated hemoglobin.

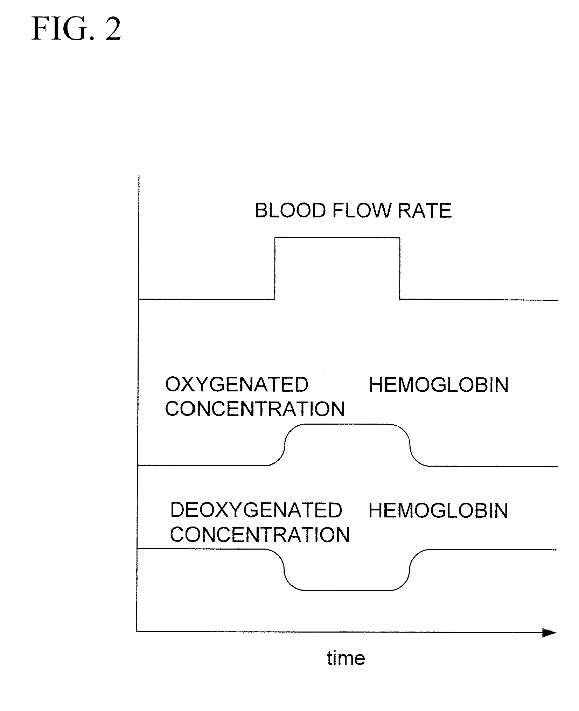

Fig. 2 is a diagram illustrating concentration changes of oxygenated hemoglobin and deoxygenated hemoglobin with changes in a blood flow rate.
As illustrated in Fig. 2, as the blood flow rate increases, the concentration of oxygenated hemoglobin increases, and the concentration of deoxygenated hemoglobin decreases.
This principle makes it possible to detect a change in the blood flow rate from the concentration changes of oxygenated hemoglobin and deoxygenated hemoglobin, and such detection can be applied to examinations for brain functions.

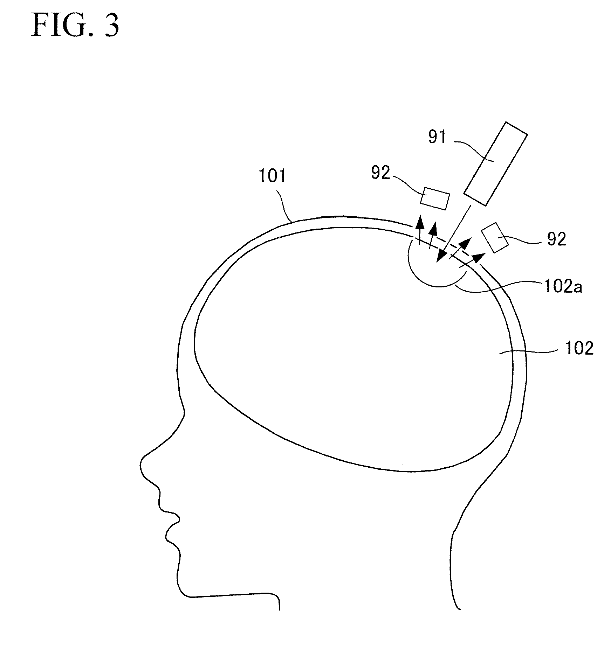

Fig. 3 is a diagram illustrating a measurement method for a brain function using near-infrared light.
As illustrated in Fig. 3, an infrared light emitting element 91 is placed on a specified region of a brain 102 of a subject 101 to be examined, for example, on the visual cortex. Then, the infrared light emitting element 91 applies infrared light. The applied infrared light is scattered at a region (visual cortex) 102a located near the surface of the brain 102, and the scattered light is detected by light receiving elements 92.
By giving a light stimulation to the eyes, for example, in this state and detecting whether or not the blood flow in the visual cortex changes in response to the stimulation, it is possible to examine a presence of abnormality in the visual function of an infant who does not understand language. In addition, if visual abnormality is found, it is possible to examine whether the cause originates simply from the eyes or from a nervous system.

However, in the case where a light-emitting diode is used as the infrared light emitting element 91 in a conventional examination apparatus, the irradiation intensity of the light-emitting diode sometimes varies over time because of the temperature dependence.

Such output variation of the light-emitting diode depending on a temperature change occurs due to a change of probe temperature caused by body temperature after the probe is attached to the body, or by changes of the body temperature during measurement, which sometimes hinders detection of changes in the blood flow.

In this respect, there has been proposed a method in which the temperature dependent characteristic of the emission intensity of the light-emitting diode is measured in advance, and current supplied to the light-emitting diode is controlled in accordance with the temperature of the probe.

However, a probe needs to be additionally provided with peripheral circuits such as a flash memory and a control IC to implement such a function, and resultantly becomes heavy.

Here, a probe is attached to a body with an adhesive tape or the like. For this reason, a large heavy probe gives great discomfort to the skin and also causes pain in some cases.

Moreover, when the probe is used for an infant or the like, there is a problem that he/she hates the large heavy probe to be attached to him/her, and hinders a smooth examination.

Hereinafter, descriptions are provided for a near-infrared optical biosensing apparatus which is configured to prevent an error in a measurement result due to a temperature change while minimizing the probe to the extent possible, and therefore is capable of conducting examinations without imposing a burden on the subject.

(Embodiment)

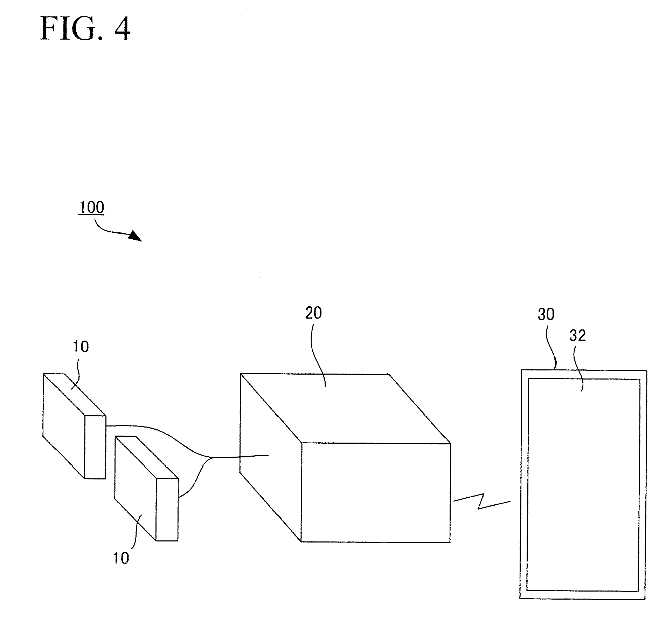

Fig. 4 is a diagram illustrating a schematic structure of a near-infrared optical biosensing apparatus according to an embodiment.

As illustrated in Fig. 4, a near-infrared optical biosensing apparatus 100 according to the embodiment includes probes 10 to be attached to a living organism. The probes 10 are connected to a control unit 20 and controlled by the control unit 20. The control unit 20 is connected to a terminal apparatus 30 through a wireless communication path.

The terminal apparatus 30 is a terminal of tablet type, which processes measurement results of the probes 10, and displays the measurement results on a display unit 32. The display unit 32 includes a touch panel in which a display screen and input means are integrated, and receives measurement conditions and measurement start and end instructions inputted by a user.

Note that although in the example of Fig. 4, the terminal apparatus 30 and the control unit 20 are separated from each other, the embodiment is not limited thereto. The terminal apparatus 30 and the control unit 20 may be integrated into one unit.

In addition, the terminal apparatus 30 is not limited to the tablet type in which the touch panel and the screen are integrated. The terminal apparatus 30 may be a computer provided with input means such as a keyboard. In that case, the connection between the terminal apparatus 30 and the control unit 20 may be a wired connection via a universal serial bus (USB) or the like, for example, or may also be a wireless connection via a wireless LAN.

Hereinafter, descriptions are further provided for a configuration of the probe 10 and the control unit 20.

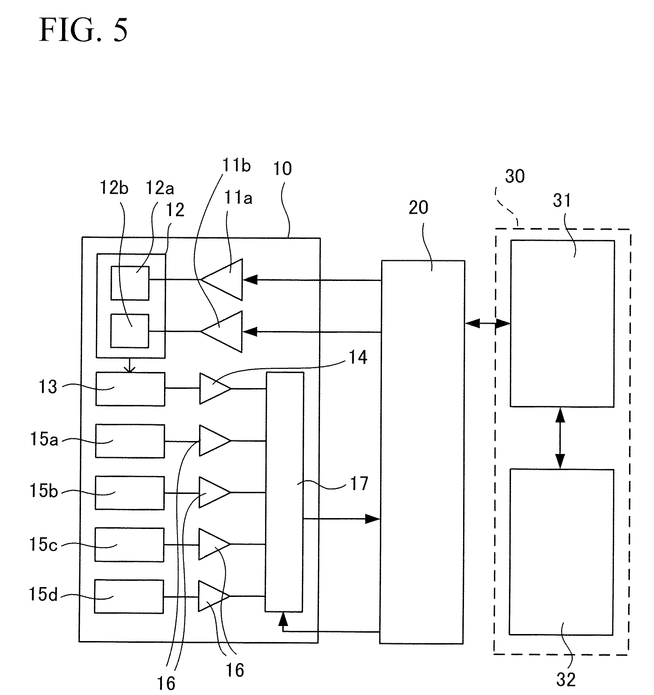

Fig. 5 is a block diagram for a control system of the near-infrared optical biosensing apparatus 100 in Fig. 4.

As illustrated in Fig. 5, the probe 10 is provided with an infrared LED 12 which emits near-infrared light. The infrared LED 12 includes a first light emitting element 12a which emits infrared light with a first wavelength $\lambda_1$ and a second light emitting element 12b which emits infrared light with a second wavelength $\lambda_2$, and is capable of outputting infrared light with any one of the two wavelengths selectively.

The first wavelength $\lambda_1$ is a wavelength (880 nm, for example) selected within a wavelength range where the absorption coefficient of oxygenated hemoglobin is larger than that of deoxygenated hemoglobin. The second wavelength $\lambda_2$ is a wavelength (730 nm, for example) selected within a wavelength range where the absorption coefficient of deoxygenated hemoglobin is relatively larger than that of oxygenated hemoglobin.

A first constant current circuit 11a is connected to the first light emitting element 12a of this infrared LED 12, and a second constant current circuit 11b is connected to the second light emitting element 12b.

These constant current circuits 11a and 11b supply drive current to the infrared light emitting elements 12a and 12b based on a control signal from the control unit 20, and the drive current supplied is a predetermined constant current independent of the temperature of the probe 10.

In this way, in the probe 10, the infrared LED 12 is driven with a constant current without performing control to make the output flux of the infrared LED 12 constant, whereby the drive circuit is downsized.

Note that measurement with three or more wavelengths may be implemented by providing the infrared LED 12 with three or more infrared light emitting elements supporting three or more wavelengths.

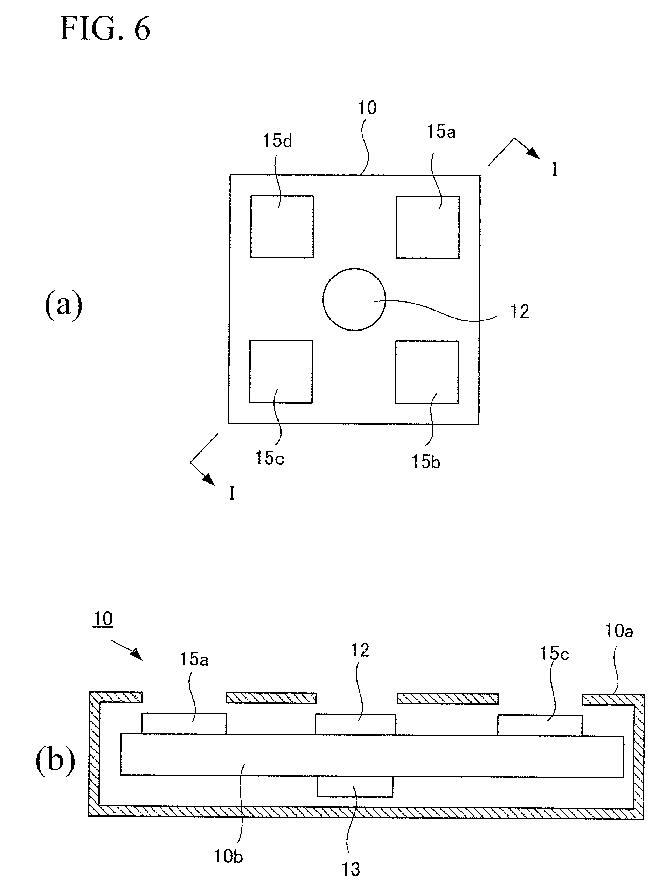

Fig. 6 (a) is a plan view illustrating a layout of the light emitting element and light receiving elements of the probe in Fig. 4, and Fig. 6(b) is a cross-sectional view illustrating the layout of the light emitting element and the light receiving elements of the probe in Fig. 4. Note that Fig. 6(b) presents a cross-sectional view taken along line I-I in Fig. 6(a).

In an example illustrated in Fig. 6(a), the infrared LED 12 is provided at the center of the probe 10. To evenly detect the light emitted by this infrared LED 12 and scattered inside the living organism, four light receiving elements 15a to 15d are arranged to surround the infrared LED 12.

In this embodiment, as illustrated in Fig. 6(b), these light receiving elements 15a to 15d each include an element such as a photodiode, and are mounted together with the infrared LED 12 on the front surface of a circuit board 10b housed in a casing 10a.

Meanwhile, an output variation detection element 13 including a photodiode or the like is mounted on the back surface of the circuit board 10b to which the infrared LED 12 is mounted. This output variation detection element 13 detects the intensity of output light of the infrared LED 12 by detecting light leaking out from the infrared LED 12 and diffused to the back side of the circuit board 10b.

As illustrated in the block diagram of Fig. 5, a detection signal of the output variation detection element 13 is amplified by an amplifier circuit 14, and then sent to the control unit 20 through a selector 17.

Detection signals of the light receiving elements 15a to 15d are amplified by amplifier circuits 16, and sent to the control unit 20 through the selector 17. The selector 17 outputs the signals of the output variation detection element 13 and the light receiving elements 15a to 15d to the control unit 20 sequentially one after another based on the control signal from the control unit 20. This makes it possible to reduce the number of transfer paths and make thin a wire connecting between the main unit 20 and the probe 10, reducing a burden on the subject when the probe 10 is attached.

Note that the output variation detection element 13 and the light receiving elements 15a to 15d may be connected in parallel to the control unit 20 through a plurality of transfer paths via the amplifier circuits 14 and 16.

The control unit 20 controls the components in the probe 10 as well as converts the detection signals of the output variation detection element 13 and the light receiving elements 15a to 15d into digital signals, and outputs the digital signals to an analysis unit 31 of the terminal apparatus 30.

The terminal apparatus 30 calculates concentrations of oxygenated hemoglobin and deoxygenated hemoglobin in the living organism in the analysis unit 31 based on detection data of the output variation detection element 13 and the light receiving elements 15a to 15d.

The analysis result of the analysis unit 31 is displayed on the display unit 32 of the terminal apparatus 30. Based on the displayed analysis result, a change in the blood flow rate in the living organism can be known.

Hereinafter, descriptions are provided for a living organism examination method using the near-infrared optical biosensing apparatus 100.

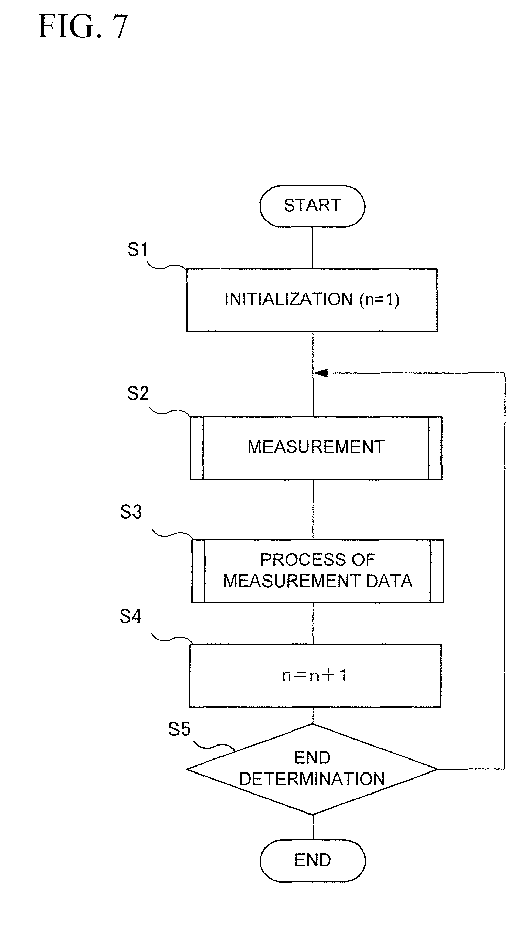

Fig. 7 is a flowchart of a measurement method using the near-infrared optical biosensing apparatus in Fig. 4.

First, before starting a measurement, the probes 10 are attached to a living organism. For example, when examining the visual function of the brain, the probes 10 need to be attached on the visual cortex of the subject.

Next, at step S1 in Fig. 7, the control unit 20 of the near-infrared optical biosensing apparatus 100 sets a measurement number n to 1 (initialization).

Next the processing proceeds to step S2, and the first (n = 1) measurement process is performed. The first measurement process measures reference values for oxygenated hemoglobin concentration and deoxygenated hemoglobin concentration. The reference values are values of the living organism at the ordinary time before various kinds of stimulation are imposed on the living organism and serves as references to be hereafter compared to measurement data to calculate concentration changes in hemoglobin.

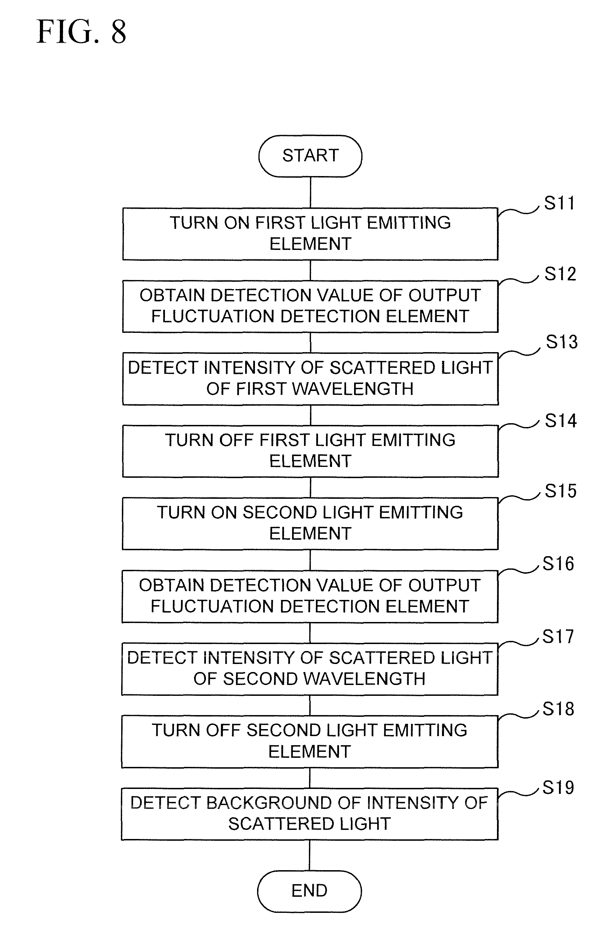

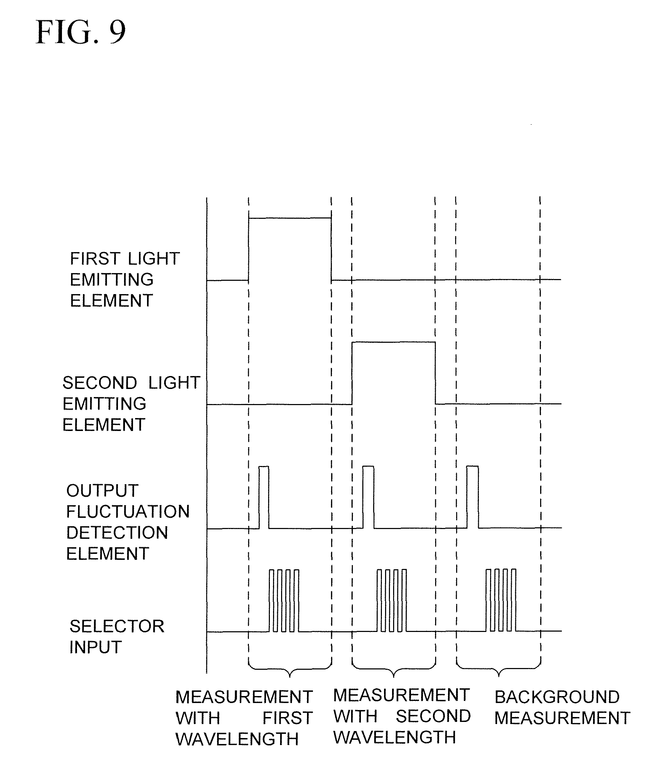

The measurement method is described specifically, referring to Fig. 8. Fig. 8 is a flowchart of a measurement process (step S2) in Fig. 7. In addition, Fig. 9 is a timing chart illustrating operations of the probe 10 and the control unit 20 along with the process in Fig. 8.

At step S11 in Fig. 8, the control unit 20 operates the first constant current circuit 11a of the probe 10 to turn on the first light emitting element 12a.

Next, the process proceeds to step S12, and the control unit 20 obtains a detection value of the output variation detection element 13.

Then, the process proceeds to step S13, and the control unit 20 obtains detection results of the four light receiving elements 15a to 15d, while switching the selector 17 by sending the control signal to the selector 17.

After that, the process proceeds to step S14, and the control unit 20 turns off the first light emitting element 12a by giving a control signal to the first constant current circuit 11a.

Through these steps, the measurement of the intensity of scattered light using the first wavelength is completed.

Next, through steps S15 to S18, a measurement using the second light emitting element 12b is performed.

At step S15, the control unit 20 turns on the second light emitting element 12b by giving a control signal to the second constant current circuit 11b of the probe 10.

Next, the process proceeds to step S16, and the control unit 20 obtains a detection value of the output variation detection element 13. From this process, the reference value of output of the second light emitting element 12b is obtained.

Then, the process proceeds to step S17, and the control unit 20 obtains detection results of the four light receiving elements 15a to 15d, while switching the selector 17 by sending a control signal to the selector 17.

After that, the process proceeds to step S18, and the control unit 20 turns off the second light emitting element 12b by stopping current output of the second constant current circuit 11b.

After that, at step S19, a background measurement is performed. In this background measurement, detection values of the output variation detection element 13 and the light receiving elements 15a to 15d are obtained while the first light emitting element 12a and the second light emitting element 12b are off.

The detection values of the light receiving elements 15a to 15d obtained at steps S13 and S19 are integrated (or averaged), and then are used for the subsequent calculations.

Through steps S11 to S19 described above, the measurement process is completed.

Next, the processing proceeds to step S3 in Fig. 7, and the analysis unit 31 calculates concentration changes in the oxygenated hemoglobin and the deoxygenated hemoglobin. Here, the analysis unit 31 calculates concentration change amounts of the oxygenated hemoglobin and the deoxygenated hemoglobin if the measurement number n is 2 or larger.

Here, the change amounts are calculated using the following formulae based on Lambert-Beer's law.

$$\Delta C_{oxy}D = \frac{\varepsilon_{deoxyL2}\log\left(\frac{I_{oL1-n}}{I_{oL1-base}} \times \frac{I_{iL1-base}}{I_{iL1-n}}\right) - \varepsilon_{deoxyL1}\log\left(\frac{I_{oL2-n}}{I_{oL2-base}} \times \frac{I_{iL2-base}}{I_{iL2-n}}\right)}{\varepsilon_{oxyL2} \times \varepsilon_{deoxyL1} - \varepsilon_{deoxyL2} \times \varepsilon_{oxyL1}}$$

$$\Delta C_{deoxy}D = \frac{\varepsilon_{oxyL1}\log\left(\frac{I_{oL2-n}}{I_{oL2-base}} \times \frac{I_{iL2-base}}{I_{iL2-n}}\right) - \varepsilon_{oxyL2}\log\left(\frac{I_{oL1-n}}{I_{oL1-base}} \times \frac{I_{iL1-base}}{I_{iL1-n}}\right)}{\varepsilon_{oxyL2} \times \varepsilon_{deoxyL1} - \varepsilon_{deoxyL2} \times \varepsilon_{oxyL1}}$$

$\Delta C_{oxy}$ : Concentration change amount of oxygenated hemoglobin

$\Delta C_{deoxy}$ : Concentration change amount of deoxygenated hemoglobin
D : Optical path length of infrared light (which is determined by the depth to which the infrared light penetrates in the living organism)
$I_{oL1-base}$ : First measurement value of the light receiving element with the first wavelength (reference value)
$I_{oL1-n}$ : n-th measurement value of the light receiving element with the first wavelength
$I_{oL2-base}$ : First measurement value of the light receiving element with the second wavelength (reference value)
$I_{oL2-n}$ : n-th measurement value of the light receiving element with the second wavelength
$I_{iL1-base}$ : First measurement value of the output variation detection element with the first wavelength (reference value)
$I_{iL1-n}$ : n-th measurement value of the output variation detection element with the first wavelength
$I_{iL2-base}$ : First measurement value of the output variation detection element with the second wavelength (reference value) $I_{iL2-n}$ : n-th measurement value of the output variation detection element with the second wavelength
$\varepsilon_{oxyL1}$ : Molar absorption coefficient of oxygenated hemoglobin with the first wavelength
$\varepsilon_{oxyL2}$ : Molar absorption coefficient of oxygenated hemoglobin with the second wavelength
$\varepsilon_{deoxyL1}$ : Molar absorption coefficient of deoxygenated hemoglobin with the first wavelength
$\varepsilon_{deoxyL2}$ : Molar absorption coefficient of deoxygenated hemoglobin with the second wavelength

In the above formulae, the measurement value of the light receiving element is divided by the measurement value of the output variation detection element, and thereby is corrected for an output variation component caused by a temperature change of the infrared LED 12.

This eliminates the need for a circuit to control output of the infrared LED 12 and makes it possible to downsize the probe 10.

After that, the analysis unit 31 displays on the display unit 32 the oxygenated hemoglobin concentration and the deoxygenated hemoglobin concentration calculated in the above method.

After that, at step S4, the counter n for the measurement number is incremented by 1.

At step S5, the control unit 20 determines whether or not to terminate the measurement.

If the control unit 20 determines at step S5 that the measurement is not to be terminated (NO), the processing proceeds to step S2 and it repeats the measurement process and the measurement data process.

On the other hand, if the control unit 20 determines at step S5 that the measurement is to be terminated (YES), it terminates the measurement.

While the measurement process as above is repeated, light is shone as visual stimulation on the eyes, and the change in the blood flow rate in the brain can be detected from the concentration changes in the hemoglobin caused by the visual stimulation.

Since the infrared optical biosensing apparatus according to this embodiment drives the infrared LED 12 only with the constant current circuits 11a and 11b without controlling the optical output of the infrared LED 12 of the probe 10, it is possible to downsize the probe 10. This makes it possible to conduct examinations using infrared light in the state where a burden on the subject is small.

Although the case of examining the visual cortex of the brain is illustrated in the above descriptions, the embodiment is not limited thereto. For example, it is possible to examine brain functions for the auditory sense or the olfactory sense, for example, by attaching the probe 10 to the region responsible for the auditory sense or the olfactory sense in the brain of the subject, and conducting the other necessary things.

(Modifications of Probe)

Hereinafter, descriptions are provided for modifications for the arrangement of the output variation detection element 13 of the probe 10.

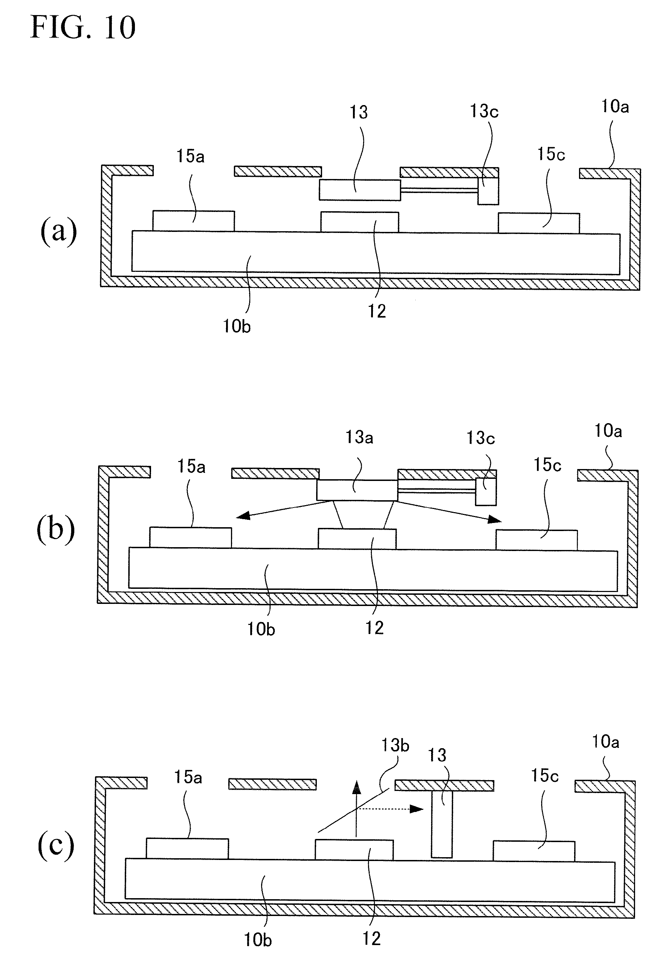

Figs. 10(a) to 10(c) are diagrams illustrating layouts of an output variation detection element 13 according to the modifications.

In a modification illustrated in Fig. 10(a), the output variation detection element 13 is arranged above the surface of a circuit board 10b. In the step of measuring the intensity of output light of an infrared LED 12, this output variation detection element 13 is positioned in front of the infrared LED 12 by a driver unit 13c and directly measures the intensity of the output light of the infrared LED 12.

In the step of applying the infrared light to a living organism, the driver unit 13c retracts the output variation detection element 13.

In this modification, the output variation detection element 13 is directly irradiated with emitted light of the infrared LED 12, and thus can measure the output of the infrared LED 12 with relatively high accuracy.

In a modification illustrated in Fig. 10(b), the output variation detection element 13 is replaced with a reflection mirror 13a, which is arranged above the front surface of a circuit board 10b. This reflection mirror 13a is positioned in front of an infrared LED 12 by a driver unit 13c. Infrared light emitted from the infrared LED 12 is reflected by the reflection mirror 13a and detected by light receiving elements 15a to 15d.

In this modification, the intensity of output light of the infrared LED 12 is detected based on output of the light receiving elements 15a to 15d. When the infrared light is applied to a living organism, the driver unit 13c retracts the reflection mirror 13a.

In this modification, the probe 10 can be simplified because a dedicated output variation detection element 13 does not need to be provided. In addition, a cable to connect the probe 10 and the control unit 20 can be thinned since there is no need for a wire to transfer a signal of the output variation detection element 13.

In a modification illustrated in Fig. 10(c), a half mirror 13b is arranged above an infrared LED 12, and infrared light reflected by the half mirror 13b is detected by an output variation detection element 13.

In this modification, it is possible to apply the infrared light to a measurement subject and simultaneously measure the intensity of output light of the infrared LED 12 with the output variation detection element 13.

Hereinafter, descriptions are provided for other applications of the near-infrared optical biosensing apparatus illustrated in Fig. 4.

(Measurement of Blood Sugar Level)

The near-infrared optical biosensing apparatus 100 is applicable to measurement of a blood sugar level in blood of the subject. In this case, infrared light with a wavelength around 1500 nm is emitted by an infrared LED 12, for example. Since the infrared light with this wavelength has a characteristic of being absorbed by glucose (sugar) in blood, it is possible to measure the blood sugar level from the intensity of scattered light coming back from the inside of the body.

According to this application, it is possible to measure the blood sugar level without damaging the subject's skin.

(Measurement of Body Fat)

The near-infrared optical biosensing apparatus 100 is also applicable to measurement of a body fat. In this case, near-infrared light with a wavelength around 950 nm, at which the absorption level of body fat is high, is applied from the infrared LED 12 to an upper arm of the subject, for example. From the intensity of the scattered light coming back from the inside of the body, it is possible to measure the percent of body fat inside the body.

(Application for Food Inspection)

In addition, the near-infrared optical biosensing apparatus 100 may be used for nondestructive measurements of sugar and fat content of food and agricultural products.

**Claims**

1.  An infrared optical biosensing apparatus comprising:

    a probe including an infrared light emitting element which emits infrared light, an output variation detection element which detects output variation of the infrared light emitting element, and a light receiving element which detects the infrared light scattered in a living organism; and
    an operation unit which calculates information on the living organism based on output data of the light receiving element, wherein
    the operation unit performs correction of a detection result of the light receiving element in accordance with a detection result of the output variation detection element, and obtains information on inside of the living organism.

2.  The infrared optical biosensing apparatus according to claim 1, wherein the infrared light emitting element is a light-emitting diode.

3.  The infrared optical biosensing apparatus according to claim 2, wherein
    the probe further includes a drive circuit which supplies drive current to the infrared light emitting element, and
    the infrared light emitting element emits light with constant drive current independent of temperature of the infrared light emitting element.

4.  The infrared optical biosensing apparatus according to any one of claim 3, wherein the operation unit performs the correction for an output variation component of the output variation detection element by dividing a detection value of the light receiving element by a detection value of the output variation detection element.

5.  The infrared optical biosensing apparatus according to claim 4, wherein
    a plurality of the light receiving elements are provided around the infrared light emitting element, and
    the probe further includes a selector circuit which transfers detection signals of the light receiving elements in sequence in a time-division manner.

6.  A probe of an infrared optical biosensing apparatus, comprising:

    an infrared light emitting element which emits infrared light;
    an output variation detection element which detects output variation of the infrared light emitting element;
    a light receiving element which detects the infrared light scattered in a living organism; and
    a drive circuit which supplies drive current to the infrared light emitting element, wherein
    the drive circuit supplies constant drive current to the infrared light emitting element independently of temperature.

7.  The probe of the infrared optical biosensing apparatus, according to claim 6, wherein the infrared light emitting element is a light-emitting diode.

8.  The probe of the infrared optical biosensing apparatus, according to claim 7, wherein
    a plurality of the light receiving elements are provided around the infrared light emitting element, and
    the probe further includes a selector circuit which transfers detection signals of the light receiving elements in sequence in a time-division manner.

# FIG. 1

# FIG. 2

# FIG. 3

# FIG. 4

# FIG. 5

# FIG. 6

(a)

(b)

# FIG. 7

START

S1 — INITIALIZATION (n=1)

S2 — MEASUREMENT

S3 — PROCESS OF MEASUREMENT DATA

S4 — $n = n + 1$

S5 — END DETERMINATION

END

# FIG. 8

START

TURN ON FIRST LIGHT EMITTING
ELEMENT                                    S11

OBTAIN DETECTION VALUE OF OUTPUT
FLUCTUATION DETECTION ELEMENT              S12

DETECT INTENSITY OF SCATTERED LIGHT
OF FIRST WAVELENGTH                        S13

TURN OFF FIRST LIGHT EMITTING
ELEMENT                                    S14

TURN ON SECOND LIGHT EMITTING
ELEMENT                                    S15

OBTAIN DETECTION VALUE OF OUTPUT
FLUCTUATION DETECTION ELEMENT              S16

DETECT INTENSITY OF SCATTERED LIGHT
OF SECOND WAVELENGTH                       S17

TURN OFF SECOND LIGHT EMITTING
ELEMENT                                    S18

DETECT BACKGROUND OF INTENSITY OF
SCATTERED LIGHT                            S19

END

# FIG. 9

FIRST LIGHT
EMITTING
ELEMENT

SECOND LIGHT
EMITTING
ELEMENT

OUTPUT
FLUCTUATION
DETECTION
ELEMENT

SELECTOR
INPUT

MEASUREMENT
WITH FIRST
WAVELENGTH

MEASUREMENT
WITH SECOND
WAVELENGTH

BACKGROUND
MEASUREMENT

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005111165 A
- JP 2006017084 A
- JP 2008178563 A